# EUROPEAN PATENT APPLICATION

(11) **EP 4 796 043 A1**
(43) Date of publication of application: **26.08.2026**
(21) Application number: 24880138.3
(22) Date of filing: 17.10.2024
(51) Int. Cl.: A61B 6/03, A61B 6/00, A61B 6/40, A61B 6/51

(54) **CT IMAGING APPARATUS**

(30) Priority: 17.10.2023 KR 20230138251
(71) Applicant: Vatech Co., Ltd., Hwaseong-si, Gyeonggi-do 18449 (KR); Vatech Ewoo Holdings Co., Ltd., Hwaseong-si, Gyeonggi-do 18449 (KR)
(72) Inventor: PARK, Chul Kyu, Hwaseong-si, Gyeonggi-do 18449 (KR); PARK, Ju Eon, Hwaseong-si, Gyeonggi-do 18449 (KR); LIM, Sung Hwan, Hwaseong-si, Gyeonggi-do 18449 (KR)
(74) Representative: Lorenz & Kollegen
(86) International application number: PCT/KR2024/015747
(87) International publication number: WO 2025/084801

(57) **Abstract**

Provided is a CT imaging apparatus capable of solving the problem in which CT numbers of left/right symmetrical anatomical structures in a reconstructed CT image differ from each other. The CT imaging apparatus according to the present invention comprises: an imaging unit including an X-ray generator and an X-ray detector that face each other with an object to be imaged therebetween; a rotation unit for rotating the X-ray generator and the X-ray detector in opposite directions around a rotation shaft between the X-ray generator and the X-ray detector; and a control unit, which controls the imaging unit and the rotation unit for X-ray imaging the object to be imaged, wherein the X-ray imaging includes first imaging in which an X-ray beam emitted from the X-ray generator rotates around the rotation shaft while being biased to either of the left side or the right side from the center of the object to be imaged, and second imaging in which the X-ray beam rotates around the rotation shaft while being biased to the other side of the left side or right side of the rotation shaft.

## Description

### Technical Field

The present disclosure relates to an X-ray Computed Tomography (CT) imaging apparatus. More particularly, the present disclosure relates to a cone-beam CT imaging apparatus.

### Background Art

In the medical field, an X-ray imaging apparatus refers to an apparatus configured to detect X-rays transmitted through a body part by using an X-ray detector and to construct an X-ray image of the body part on the basis of detected electrical signals. X-rays are attenuated at different attenuation rates according to materials along a propagation path of the X-rays, and are converted into electrical signals by a photoelectric effect when the X-rays reach an X-ray detector. The X-ray imaging apparatus provides information about an inner portion of an imaging target as an X-ray image by using electrical signals reflecting cumulative attenuation amounts along the propagation path of the X-rays.

In the field of dental treatment, a Computed Tomography (CT) image accurately and clearly displays not only a three-dimensional X-ray image of major regions of interest of a patient, including dentition, a temporomandibular joint, and a portion of the head, but also a tomographic image corresponding to a position and a direction desired by a user, so that the CT image is utilized in the field requiring high precision such as an implant procedure and so on. An X-ray CT imaging apparatus acquires X-ray projection data acquired by imaging an imaging target region from multiple angles and reconstructs the X-ray projection data to provide a tomographic image and a three-dimensional image of the imaging target region. For this purpose, the X-ray CT imaging apparatus includes an X-ray generator, an X-ray detector disposed to face the X-ray generator with an imaging target interposed therebetween, a rotation unit configured to rotate the X-ray generator and the X-ray detector in opposite directions, and an image reconstruction unit configured to reconstruct a CT image from X-ray projection data detected by the X-ray detector.

For X-ray CT imaging, the X-ray generator and the X-ray detector are configured to be rotated in opposite directions within a predetermined angular range around a rotation shaft disposed therebetween and are configured to acquire X-ray projection data of an imaging target region that is a Field Of View (FOV) from multiple angles. Generally, it is known that X-ray projection data acquired over an angular range equal to or more than 180 degrees for each portion of the FOV is required for CT image reconstruction. Accordingly, in a case of a full-beam type Cone-Beam CT (CBCT) imaging apparatus in which an X-ray beam covers an entire width (a size in a direction perpendicular to a rotation shaft and a traveling direction of an X-ray beam) of an FOV, an imaging unit including an X-ray generator and an X-ray detector acquires X-ray imaging data while rotating around the FOV through a predetermined angle, for example, equal to or larger than 180 degrees.

In a half-beam type CT imaging apparatus in which an X-ray beam covers one half of the width of the FOV in order to image a wide FOV using an X-ray detector having a limited size, an imaging unit acquires X-ray projection data while rotating the FOV through an angle at least twice a rotation angle of the full-beam type CT imaging apparatus, for example, equal to or larger than 360 degrees. However, in the half-beam type CT imaging apparatus, since the X-ray detector is biased toward one side of the FOV during an acquisition process of projection data and an X-ray beam exhibits left-right asymmetry, truncation artifact occurs, so that a CT number (HU, Hounsfield Unit), which is an intrinsic value of left-right symmetrical anatomical structures within the imaging target region, differs from each other.

### Disclosure

### Technical Problem

Accordingly, the present disclosure has been made keeping in mind the above problems occurring in the related art, and an objective of the present disclosure is to provide a CT imaging apparatus capable of solving a problem in which CT numbers of left/right symmetrical anatomical structures become different from each other while using X-ray detectors having the same size and the same number as those of a conventional half-beam type CT imaging apparatus.

### Technical Solution

In order to solve the problems described above, in the present disclosure, there is provided a Computed Tomography (CT) imaging apparatus including: an imaging unit including an X-ray generator and an X-ray detector that face each other with an imaging target interposed therebetween; a rotation unit configured to rotate the X-ray generator and the X-ray detector in opposite directions around a rotation shaft disposed between the X-ray generator and the X-ray detector; and a control unit configured to control the imaging unit and the rotation unit so as to perform X-ray imaging of the imaging target, wherein the X-ray imaging includes: first imaging in which an X-ray beam emitted from the X-ray generator rotates around the rotation shaft in a state in which the X-ray beam is biased toward one of left and right sides of the rotation shaft from a center of the imaging target; and second imaging in which the X-ray beam rotates around the rotation shaft in a state in which the X-ray beam is biased toward a remaining one of the left and right sides of the rotation shaft.

The control unit may be configured to move the rotation shaft between the first imaging and the second imaging.

The control unit may be configured to move the rotation shaft along different first and second trajectories during the first imaging and the second imaging, respectively.

The first trajectory and the second trajectory may be arc trajectories following concentric circles having different radii.

The CT imaging apparatus may further include a connection trajectory that connects the first trajectory and the second trajectory, wherein the control unit may be configured to move the rotation shaft along the connection trajectory between the first imaging and the second imaging.

The X-ray generator and the X-ray detector may be configured to be rotated in different directions with respect to the imaging target during the first imaging and the second imaging, respectively.

The X-ray detector may be configured to acquire a plurality of X-ray projection data during each of the first imaging and the second imaging, wherein the X-ray projection data of the first imaging and the X-ray projection data of the second imaging may correspond to each other in a one-to-one manner.

The X-ray beam may pass through the center of the imaging target during the first imaging and the second imaging.

According to an aspect of the present disclosure, there is provided a CT imaging apparatus including: an imaging unit including an X-ray generator and an X-ray detector that face each other with an imaging target interposed therebetween; a rotation unit configured to rotate the X-ray generator and the X-ray detector in opposite directions around a rotation shaft disposed between the X-ray generator and the X-ray detector; and a control unit configured to control the imaging unit and the rotation unit so as to perform X-ray imaging of the imaging target, wherein the X-ray imaging includes: first half-beam imaging in which an X-ray beam emitted from the X-ray generator penetrates one of left and right sides from a center of the imaging target and rotates around the rotation shaft; and second half-beam imaging in which the X-ray beam penetrates a remaining one of the left and right sides from the center of the imaging target and rotates around the rotation shaft.

### Advantageous Effects

In the CT imaging apparatus according to the present disclosure, there is an effect that a more accurate CT image is capable of being provided by solving the problem in which CT numbers of left-right symmetrical anatomical structures become different from each other in a reconstructed CT image while using X-ray detectors having the same size and the same number as those of a conventional half-beam type CT imaging apparatus.

### Description of Drawings

FIG. 1 is a view illustrating a configuration of a Computed Tomography (CT) imaging apparatus according to an embodiment of the present disclosure.
FIG. 2 shows an imaging sequence of a conventional half-beam CT imaging apparatus.
FIG. 3 shows a CT imaging sequence of the CT imaging apparatus according to an embodiment of the present disclosure.
FIG. 4 shows a movement trajectory of a rotation shaft of an imaging unit when the CT imaging sequence of the CT imaging apparatus is performed according to an embodiment of the present disclosure.
FIG. 5 shows a position change of the rotation shaft of the imaging unit when the CT imaging sequence of the CT imaging apparatus is performed according to an embodiment of the present disclosure.
FIG. 6 shows a change of a half-beam according to a movement of a detector when the CT imaging sequence of the CT imaging apparatus is performed according to an embodiment of the present disclosure.
FIG. 7 is a view illustrating scan regions for respective sections of an imaging sequence in a CT image reconstructed by the CT imaging apparatus according to an embodiment of the present disclosure.
FIG. 8 shows a comparison between a CT image acquired by the CT imaging apparatus according to an embodiment of the present disclosure and an image acquired by a conventional method.
FIG. 9 shows a comparison between a tomographic image acquired by the CT imaging apparatus according to an embodiment of the present disclosure and an image acquired by the conventional method.

### Mode for Invention

Hereinafter, various embodiments of the present disclosure will be described with reference to the drawings. Through the embodiments, a technical concept of the present disclosure may be more clearly understood. The present disclosure is not limited to the embodiments described below.

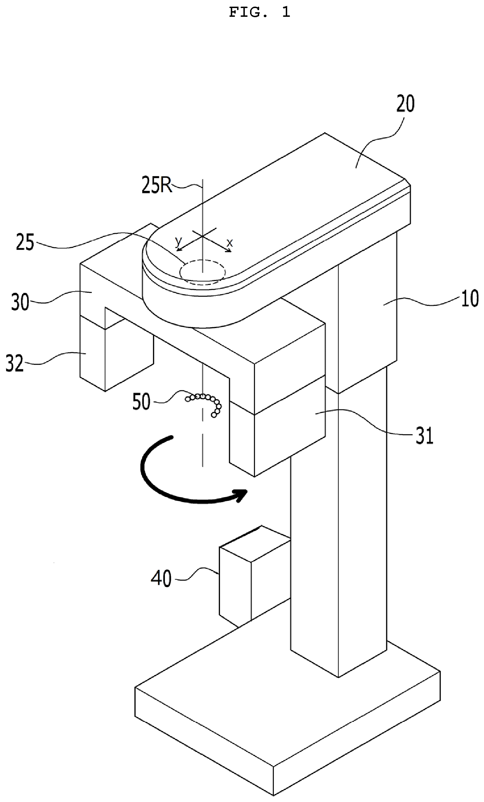

FIG. 1 is a view illustrating a configuration of a Computed Tomography (CT) imaging apparatus according to an embodiment of the present disclosure.

Similar to a conventional dental X-ray imaging apparatus, a CT imaging apparatus according to the present embodiment includes a column erected vertically from a base placed on a floor and a lifting unit 10 configured to be moved upward and downward along the column according to a height of a subject. A rotation arm support unit 20 is connected to a first side of the lifting unit 10. A rotation unit 30 is connected to a lower portion of the rotation arm support unit 20 such that the rotation unit 30 is capable of being rotated. The rotation unit 30 includes a generator unit 32 disposed at one side of the rotation unit 30 with a rotation shaft 25C interposed between the generator unit 32 and a detector unit 31, and includes the detector unit 31 disposed at the other side of the rotation unit 30 so as to face the generator unit 32. An X-ray generator configured to emit an X-ray beam having predetermined widths in upper, lower, left, and right directions from an X-ray emission focal spot F is disposed in the generator unit 32, and an X-ray detector D configured to receive the X-ray beam is disposed in the detector unit 31. During CT imaging, a portion of the head of the subject including a dental arch 50 of the subject is disposed at a predetermined alignment position between the generator unit 32 and the detector unit 31, and a relative position and a relative direction of the detector D within the detector unit 31 and a relative position and a relative direction of the X-ray generator within the generator unit 32 are maintained constant.

A rotation driving unit 25 configured to connect the rotation unit 30 and the rotation arm support unit 20 and to rotate the rotation unit 30 around the rotation shaft 25C by using power is provided between the rotation unit 30 and the rotation arm support unit 20. The rotation driving unit 25 serves to rotate the rotation unit 30 by a desired angle when X-ray projection data is acquired from multiple angles in order to acquire a CT image of the portion of the head of the subject including the dental arch 50 of the subject. In addition, the rotation driving unit 25 may be configured to move a position of the rotation shaft 25C on a two-dimensional plane (an XY plane) while the rotating driving unit 25 rotates the rotation unit 30.

Meanwhile, the CT imaging apparatus according to the present embodiment includes a control unit 40, and z, a movement of the rotation shaft 25C, and operations of the X-ray generator and the detector D are controlled by the control unit 40. The control unit 40 stores imaging sequence information preset for each option of an imaging target region (FOV, Field of View), and is configured to control, according to a time line, a position of the rotation shaft 25C, an angle of the rotation unit 30, an operation of the X-ray generator, and an acquisition of X-ray projection data through the detector D. In addition, the control unit 40 may include an image reconstruction unit configured to reconstruct a CT image by using X-ray projection data of multiple frames acquired through the imaging sequence. The control unit 40 may be disposed inside or outside the CT imaging apparatus according to the present embodiment. Furthermore, in the control unit 40, a portion performing a control function and a portion performing an image reconstruction and so on may be separately disposed.

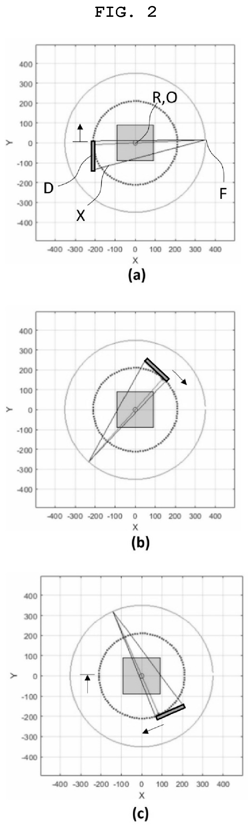

FIG. 2 shows an imaging sequence of a conventional half-beam CT imaging apparatus.

In the drawing, F denotes a focal spot of an X-ray generator, D denotes a detector, and X denotes an emission range of an X-ray beam. Hereinafter, for convenience of description, the X-ray generator and the detector disposed so as to face each other with a relative position fixed will be collectively referred to as an imaging unit. R denotes a rotation shaft of the imaging unit, and the rotation shaft R corresponds to the rotation shaft 25C of the rotation unit 30 illustrated in FIG. 1. O denotes a center of an imaging target region.

FIG. 2(a), FIG. 2(b), and FIG. 2(c) illustrate positions and directions of the imaging unit according to a time sequence when a conventional half-beam CT imaging sequence is performed. Generally, when the conventional half-beam CT imaging sequence is performed, the rotation shaft R of the imaging unit is positioned at a center of the imaging target region (FOV, Field of View) from which a CT image is to be acquired. Although the FOV is a cylindrical shape typically, a quadrangle at a central portion in the drawing schematically represents the FOV. During the imaging sequence, the rotation shaft R of the imaging unit is positioned at the center O of the FOV.

FIG. 2(a) is a view illustrating a state in which a half-beam biased toward a left side from the center of the FOV on the basis of a traveling direction of the X-ray beam X starts rotating clockwise from an azimuth angle of 0 degrees of the imaging unit, FIG. 2(b) is a view illustrating a state in which the imaging unit is rotated approximately 135 degrees, and FIG. 2(c) is a view illustrating a state in which the imaging unit is rotated approximately 240 degrees. The imaging unit rotates one full revolution until the imaging unit reaches approximately 360 degrees. During this process, X-ray projection data are acquired each time a front end portion of the detector D passes through positions indicated by blue dots.

Here, from a start to an end of the CT imaging sequence, the imaging unit is rotated in a state in which a first side end in a width direction of the X-ray beam X covers the center of the FOV while a center in the width direction of the X-ray beam X is biased toward a left side with respect to the center of the FOV.

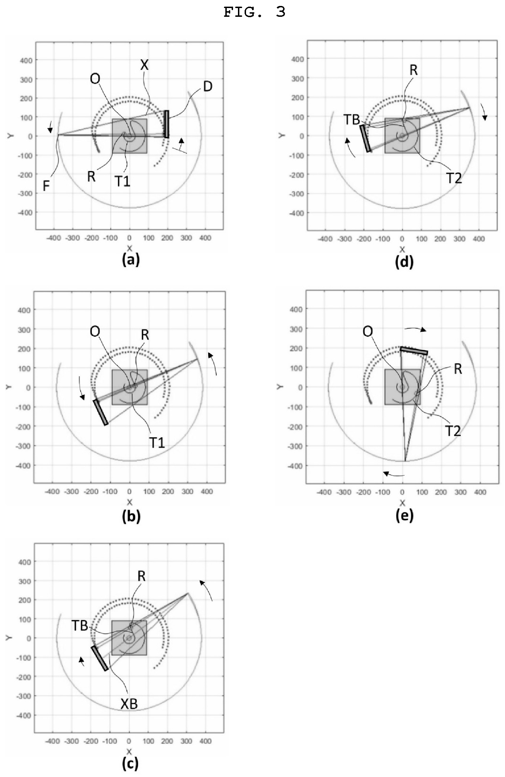

FIG. 3 shows a CT imaging sequence of the CT imaging apparatus according to an embodiment of the present disclosure.

In the drawing, F, D, X, and R respectively denote elements identical to those described with reference to FIG. 2. FIG. 3(a) to FIG. 3(e) are views illustrating angles of an imaging unit and positions of the rotation shaft R according to a time sequence. Here, O denotes a center of an imaging target region (FOV).

In FIG. 3(a), it can be seen that the imaging unit is starting to rotate counterclockwise from a direction in which an X-ray beam X is emitted from a left side toward a right side of the drawing. Here, the rotation shaft R of the imaging unit is positioned adjacent to a starting point of a first trajectory T1 having a shape of a small circular arc with respect to the center of the FOV. While the rotation shaft R passes along the first trajectory T1, the X-ray beam X is a left half-beam biased toward a left side from the center of the FOV, and a right end portion of the center in the width direction of the X-ray beam X overlaps the center of the FOV.

In FIG. 3(b), the imaging unit continues rotating counterclockwise from the state in FIG. 3(a), and it can be seen that the rotation shaft R moves and rotates along the first trajectory T1 and reaches an end portion of the first trajectory T1 having a first circular arc shape having a relatively small radius. Until this stage, the X-ray beam X maintains a left half-beam state in which the X-ray beam is biased toward the left side from the center of the FOV.

A sequence from FIG. 3(a) to FIG. 3(b) may be defined as a first partial sequence of a CT imaging sequence, i.e., a first imaging. During the first partial sequence, X-ray projection data are acquired through the imaging unit at predetermined angles.

Next, in FIG. 3(c), the rotation shaft R of the imaging unit moves and rotates on a connection trajectory TB in which the rotation shaft R transitions from the first trajectory T1 to a second trajectory T2 that has a circular arc shape having a radius relatively larger than that of the first trajectory T1, and a transition section in which the X-ray beam X transitions from the left half-beam biased toward the left side of the center of the FOV to a right half-beam biased toward a right side of the center of the FOV. During the transition section, a center line XB in the width direction of the X-ray beam X passes through the center of the FOV from left to right. During the transition section, the control unit may suspend the acquisition of X-ray projection data through the imaging unit.

A sequence from FIG. 3(d) to FIG. 3(e) may be defined as a second partial sequence of the CT imaging sequence, i.e., a second imaging. During the second partial sequence, the rotation shaft R of the imaging unit moves and rotates along the second trajectory T2, and the imaging unit rotates clockwise while acquiring X-ray projection data at predetermined angles. During the second partial sequence, the X-ray beam X is a right half-beam biased toward the right side of the center of the FOV, and a left end portion in the width direction of the X-ray beam X is maintained in a state in which the left end portion of the X-ray beam X overlaps the center of the FOV.

In the present embodiment, the second trajectory T2 has a circular arc shape of a concentric circle having a radius relatively larger than the radius of the first trajectory T1. As described above, the first trajectory T1 and the second trajectory T2 have circular arc shapes with different radii, and relative lengths of the radii may be configured independently of an order of the first and second partial sequences.

In the present embodiment, a shape formed by lines connecting an emission focal spot of the X-ray generator and both end portions of the detector and a light receiving surface of the detector is a right triangle, but the shape is not limited thereto. For example, an emission range shape of the X-ray beam formed by the emission focal spot and the detector may form an isosceles triangular shape. In this case, projection data acquired through the first and second partial sequences, i.e., projection data of the left and right sides of the FOV, become closer to complete symmetry.

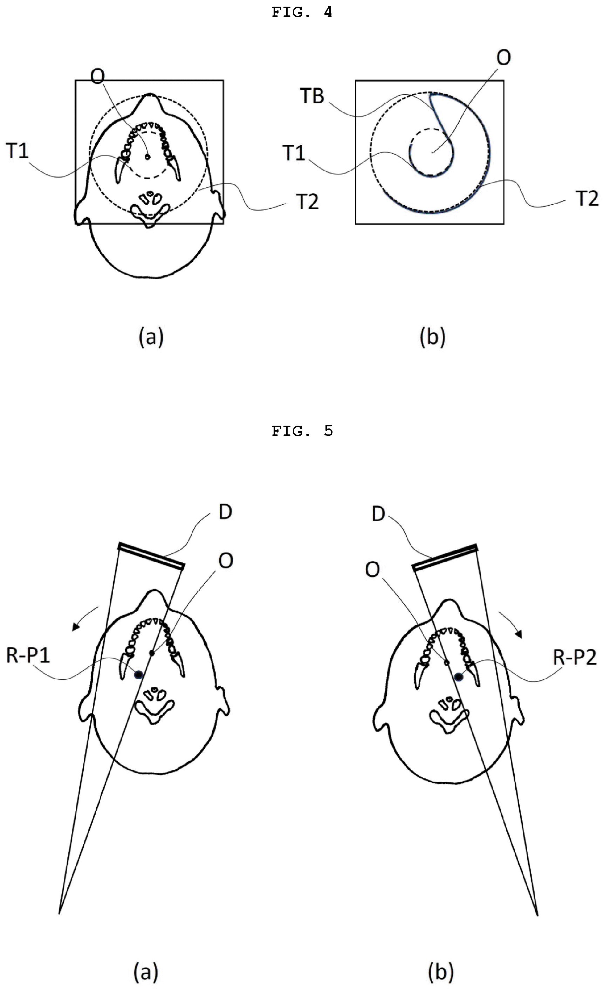

FIG. 4 shows a movement trajectory of a rotation shaft of an imaging unit when the CT imaging sequence of the CT imaging apparatus is performed according to an embodiment of the present disclosure.

With reference to the drawing, shapes and positions of the first and second trajectories T1 and T2 and the connection trajectory TB and a relationship between the trajectories T1, T2, and TB and the FOV may be examined in more detail.

FIG. 4(a) is a view illustrating a state in which a tomographic slice including a dental arch and a temporomandibular joint (TMJ) that are regions of interest in a portion of the head of a subject is disposed within an approximate imaging target region (FOV), and is a view simultaneously illustrating an arrangement of two concentric circles having different radii corresponding respectively to the first trajectory T1 and the second trajectory T2, thereby facilitating understanding of a trajectory position of the rotation shaft of the imaging unit.

FIG. 4(b) is a view illustrating, by a solid line, an actual trajectory through which the rotation shaft of the imaging unit substantially passes in the two concentric circles during the CT imaging sequence. Referring also to FIG. 3, the rotation shaft R of the imaging unit moves along the first trajectory T1 indicated by the solid line during the first partial sequence of the CT imaging sequence, and moves along the second trajectory T2 indicated by the solid line during the second partial sequence. Between the first partial sequence and the second partial sequence, the transition section in which the imaging unit moves without acquiring X-ray projection data exists. In addition, during the transition section, the rotation shaft R of the imaging unit is configured to move along the connection trajectory TB connecting a rear end portion of the first trajectory T1 and a front end portion of the second trajectory T2 to each other.

In the present embodiment, the rotation shaft R of the imaging unit may move along a section corresponding to approximately 210 degrees of rotation along the circular arc of the first trajectory T1, may move along the connection trajectory TB, and then may move along a section corresponding to approximately 210 degrees of rotation along the circular arc of the second trajectory T2. Angular ranges through which the rotation shaft R of the imaging unit rotates around the center point of the concentric circles along the first trajectory T1 and the second trajectory T2 are identical to each other, and are preferably larger than 180 degrees.

In the present embodiment, during the first partial sequence, the imaging unit emits the left half-beam and rotates counterclockwise, and the rotation shaft of the imaging unit also rotates counterclockwise along the first trajectory T1. In addition, during the second partial sequence, the imaging unit emits the right half-beam and rotates clockwise after changing a rotation direction, and the rotation shaft of the imaging unit also rotates clockwise along the second trajectory T2. The rotation direction of the imaging unit and the movement direction of the rotation shaft may be variously designed, and the rotation in mutually opposite directions during the first partial sequence and the second partial sequence is not necessarily required. However, when the imaging unit rotates while emitting a half-beam biased toward one of the left and right sides of the FOV during the first partial sequence, an overall CT imaging sequence is configured such that, during the transition section, the direction and the position are changed so that a half-beam biased toward the other side is capable of being emitted, and during the second partial sequence, the imaging unit rotates while emitting a half-beam biased toward a side different from the side used in the first partial sequence.

FIG. 5 shows a position change of the rotation shaft of the imaging unit when the CT imaging sequence of the CT imaging apparatus is performed according to an embodiment of the present disclosure.

In the drawing, FIG. 5(a) is a view illustrating a position of a half-beam with respect to the center of the FOV and a position and a rotation direction of the rotation shaft of the imaging unit when the first partial sequence is performed, and FIG. 5(b) is a view illustrating a position of a half-beam with respect to the center of the FOV and a position and a rotation direction of the rotation shaft of the imaging unit when the second partial sequence is performed.

For example, as illustrated in FIG. 5(a), when the first partial sequence is performed, the control unit of the CT imaging apparatus according to the present embodiment may rotate the imaging unit in a state R-P1 in which a left half-beam biased toward the left side with respect to the center O of the FOV is emitted and the rotation shaft R of the imaging unit is positioned at a first position P1. Next, as illustrated in FIG. 5(b), when the second partial sequence is performed, the control unit may rotate the imaging unit in a state R-P2 in which a right half-beam biased toward the right side with respect to the center O of the FOV is emitted and the rotation shaft R of the imaging unit is positioned at a second position P2. Between the first partial sequence and the second partial sequence, the rotation shaft R of the imaging unit is moved from the first position P1 to the second position P2. During the position movement of the rotation shaft R, the control unit may temporarily suspend X-ray emission and acquisition of projection data through the imaging unit. The positions P1 and P2 and the rotation direction of the rotation shaft R of the imaging unit presented in the drawing of the present embodiment are one example, and the rotation direction of the imaging unit is not limited according to the position of the rotation shaft.

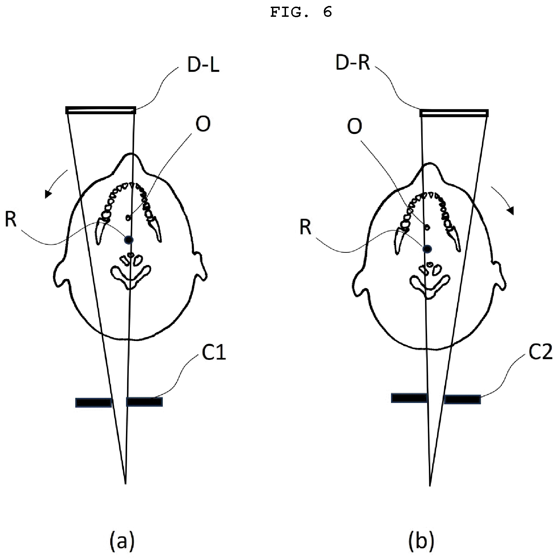

FIG. 6 shows a change of a half-beam according to a movement of a detector when the CT imaging sequence of the CT imaging apparatus is performed according to an embodiment of the present disclosure.

In the drawing, FIG. 6(a) is a view illustrating a position of a half-beam with respect to the center of the FOV and a position and a rotation direction of the rotation shaft of the imaging unit when the first partial sequence is performed, and FIG. 6(b) is a view illustrating a position of a half-beam with respect to the center of the FOV and a position and a rotation direction of the rotation shaft of the imaging unit when the second partial sequence is performed.

For example, as illustrated in FIG. 6(a), when the first partial sequence is performed, the control unit of the CT imaging apparatus according to the present embodiment may rotate the imaging unit in a state R-P in which a left half-beam biased toward the left side with respect to the center O of the FOV is emitted and the rotation shaft R of the imaging unit is stopped at one position P. Next, as illustrated in FIG. 6(b), when the second partial sequence is performed, the control unit may rotate the imaging unit in the state R-P in which a right half-beam biased toward the right side with respect to the center O of the FOV is emitted and the rotation shaft R of the imaging unit is stopped at the same position P.

When the imaging sequence is transitioned from the first partial sequence to the second partial sequence, within the detector unit 31 (see FIG. 1), the detector D may be moved in a width direction from a position D-L biased leftward with respect to an X-ray beam traveling direction to reach a position D-R biased rightward with respect to the X-ray beam traveling direction. Here, in the generator unit 32 (see FIG. 1), an X-ray collimator configured to control an emission range of an X-ray beam may be adjusted as C1 of FIG. 6(a) and C2 of FIG. 6(b) in the drawings so that the emission ranges of the X-ray beam may be configured to be matched to corresponding detector positions D-L and D-R. The position R-P and the rotation direction of the rotation shaft R of the imaging unit presented in the drawing of the present embodiment are one example, and the rotation direction of the imaging unit is not limited according to the detector positions D-L and D-R.

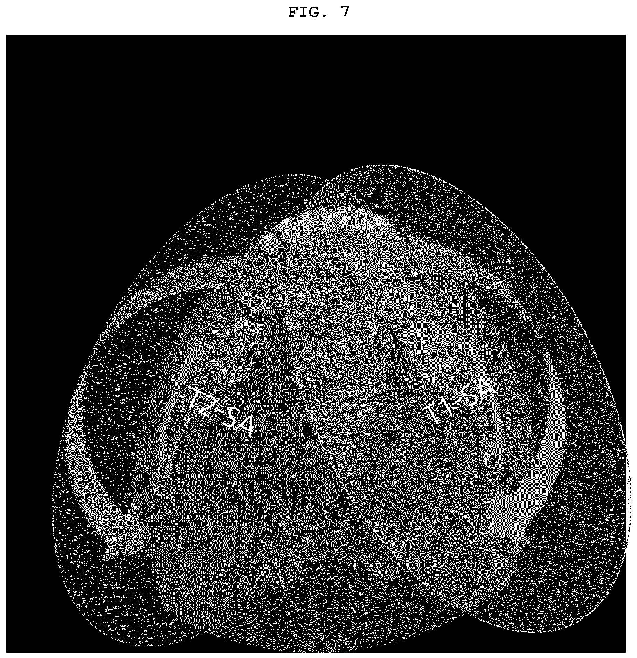

FIG. 7 is a view illustrating scan regions for respective sections of an imaging sequence in a CT image reconstructed by the CT imaging apparatus according to an embodiment of the present disclosure.

The drawing indicates major scan regions of each of two partial sequences. Through progression of the first partial sequence described above, scan data of a right region T1-SA from a center portion to a right side portion of the drawing are acquired. Furthermore, through progression of the second partial sequence, scan data of a left region T2-SA from the center portion to a left side portion of the drawing are acquired. For the right region T1-SA, a left half-beam is emitted from an inner side toward an outer side of a tooth arrangement. Furthermore, for the left region T2-SA, a right half-beam is emitted from the inner side toward the outer side of the tooth arrangement. Therefore, emission directions of X-ray beams emitted during scanning for the two regions become symmetrical to each other.

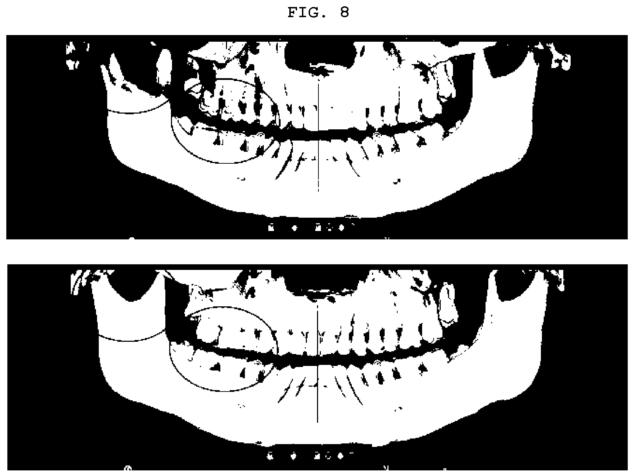

FIG. 8 shows a comparison between a CT image acquired by the CT imaging apparatus according to an embodiment of the present disclosure and an image acquired by a conventional method.

The drawing is provided to compare a result image of the CT imaging apparatus in which the new half-beam CT imaging sequence of the present disclosure is applied with an image of a conventional half-beam CT imaging apparatus. With an identical skull phantom as an imaging target, FIG. 8(a) is a view illustrating a CT image acquired by a conventional half-beam CT imaging sequence as shown in FIG. 2, and FIG. 8(b) is a view illustrating a CT image acquired by the new half-beam CT imaging sequence according to the present disclosure as shown in FIG. 3.

Portions indicated by ellipses are portions in which meaningful differences can be seen between FIG. 8(a) and FIG. 8(b). In the conventional image of FIG. 8(a), CT numbers of a left temporomandibular joint (TMJ) portion and a molar portion are distorted and are not accurately displayed compared to a right side. In the image of FIG. 8(b) according to the present disclosure, CT numbers of corresponding portions are normalized and are displayed equivalently to right-side symmetrical anatomical structures.

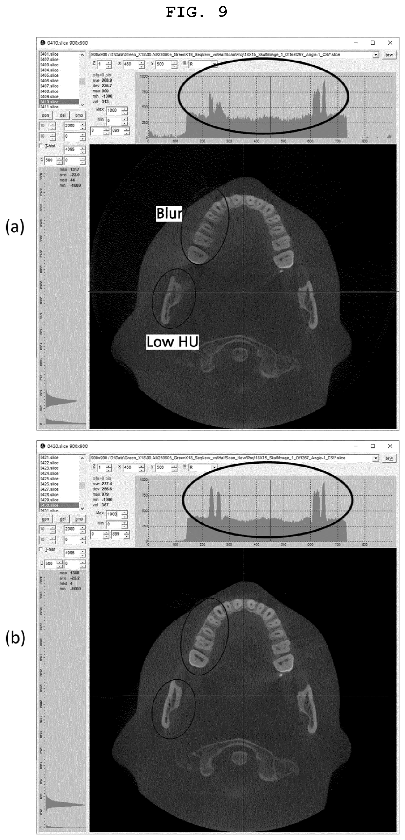

FIG. 9 shows a comparison between a tomographic image acquired by the CT imaging apparatus according to an embodiment of the present disclosure and an image acquired by the conventional method.

Similarly to FIG. 6 described above, with an identical skull phantom as an imaging target, FIG. 9(a) is a view illustrating a tomographic image acquired by a conventional half-beam CT imaging sequence as shown in FIG. 2, and FIG. 9(b) is a view illustrating a tomographic image acquired by the new half-beam CT imaging sequence according to the present disclosure as shown in FIG. 3. The oral tomographic images are reconstructed by a Compressed Sensing (CS) algorithm.

In the drawings, portions indicated by ellipses are portions in which meaningful differences can be seen between FIG. 9(a) and FIG. 9(b). In the conventional image of FIG. 9(a), CT numbers of a left temporomandibular joint (TMJ) portion and a molar portion are distorted and are not accurately displayed compared to a right side. In the image of FIG. 9(b) according to the present disclosure, CT numbers of corresponding portions are normalized and are displayed equivalently to right-side symmetrical anatomical structures. Through diagrams displayed on each tomographic image of FIG. 9(a) and FIG. 9(b), CT numbers of left and right TMJ portions and molar portions can be compared. In FIG. 9(a), a left CT number is significantly lower than a right CT number, whereas, in FIG. 9(b), left and right CT numbers show a symmetrical distribution.

As described above, by applying the new half-beam CT imaging sequence including the transition section in which the transition is performed from a left half-beam to a right half-beam or from a right half-beam to a left half-beam during progression of the CT imaging sequence and including first and second partial sequences before and after the transition section, the CT imaging apparatus may provide a more accurate CT image by correcting CT number distortion for a specific portion while using an X-ray detector having an identical size.

### INDUSTRIAL APPLICABILITY

The present disclosure may be utilized in the field of X-ray CT imaging apparatuses.

## Claims

1. A Computed Tomography (CT) imaging apparatus comprising:
an imaging unit comprising an X-ray generator and an X-ray detector that face each other with an imaging target interposed therebetween;
a rotation unit configured to rotate the X-ray generator and the X-ray detector in opposite directions around a rotation shaft disposed between the X-ray generator and the X-ray detector; and
a control unit configured to control the imaging unit and the rotation unit so as to perform X-ray imaging of the imaging target,
wherein the X-ray imaging comprises:
first imaging in which an X-ray beam emitted from the X-ray generator rotates around the rotation shaft in a state in which the X-ray beam is biased toward one of left and right sides of the rotation shaft from a center of the imaging target; and
second imaging in which the X-ray beam rotates around the rotation shaft in a state in which the X-ray beam is biased toward a remaining one of the left and right sides of the rotation shaft.

2. The CT imaging apparatus of claim 1, wherein the control unit is configured to move the rotation shaft between the first imaging and the second imaging.

3. The CT imaging apparatus of claim 1, wherein the control unit is configured to move the rotation shaft along different first and second trajectories during the first imaging and the second imaging, respectively.

4. The CT imaging apparatus of claim 3, wherein the first trajectory and the second trajectory are arc trajectories following concentric circles having different radii.

5. The CT imaging apparatus of claim 3, further comprising:
a connection trajectory that connects the first trajectory and the second trajectory,
wherein the control unit is configured to move the rotation shaft along the connection trajectory between the first imaging and the second imaging.

6. The CT imaging apparatus of claim 1, wherein the X-ray generator and the X-ray detector are configured to be rotated in different directions with respect to the imaging target during the first imaging and the second imaging, respectively.

7. The CT imaging apparatus of claim 1, wherein the X-ray detector is configured to acquire a plurality of X-ray projection data during each of the first imaging and the second imaging, and
wherein the X-ray projection data of the first imaging and the X-ray projection data of the second imaging correspond to each other in a one-to-one manner.

8. The CT imaging apparatus of claim 1, wherein the X-ray beam passes through the center of the imaging target during the first imaging and the second imaging.

9. A CT imaging apparatus comprising:
an imaging unit comprising an X-ray generator and an X-ray detector that face each other with an imaging target interposed therebetween;
a rotation unit configured to rotate the X-ray generator and the X-ray detector in opposite directions around a rotation shaft disposed between the X-ray generator and the X-ray detector; and
a control unit configured to control the imaging unit and the rotation unit so as to perform X-ray imaging of the imaging target,
wherein the X-ray imaging comprises:
first half-beam imaging in which an X-ray beam emitted from the X-ray generator penetrates one of left and right sides from a center of the imaging target and rotates around the rotation shaft; and
second half-beam imaging in which the X-ray beam penetrates a remaining one of the left and right sides from the center of the imaging target and rotates around the rotation shaft.
